# EUROPEAN PATENT APPLICATION

(11) **EP 1 378 177 A1**
(43) Date of publication of application: **07.01.2004**
(21) Application number: 02388044.6
(22) Date of filing: 02.07.2002
(51) Int. Cl.: A23K 1/16, A23K 1/175, A61K 31/355, A61K 31/375, A61K 33/04

(54) **Vitamin-containing system for stabilising the immune response of animals**

(71) Applicant: Hansen, John Erik, 4600 Koge (DK)
(72) Inventor: The designation of the inventor has not yet been filed
(74) Representative: Christensen, Bent

(57) **Abstract**

A vitamin-containing system for stabilising the external and the internal immune response of animals consists of two parts or "kits" to be used successively or separately, one mainly including biotin and the other mainly including vitamins E and C. Both kits include allicin and crushed radishes and are mixed in a carrier.

The system is used for prophylactic or curing treatment of a number of diseases in animals, especially in farming stocks.

## Description

The present invention relates to a vitamin-containing system for stabilising the immune response of animals, said system consisting of two parts or "kits" to be used either successively or separately for a prophylactic or curing treatment of a number of diseases in animals, especially in farming stocks.

The invention is based on the idea that a visual/practical evaluation is carried out of the deficiency symptoms and the unsatisfactory flourishing of the stocks whereafter said stocks are subjected to a treatment or a curing by means of a suitable vitamin-containing system which is described in greater detail below.

It is common knowledge that a wide range of diseases and health conditions apply to farming stocks, and that these diseases and health conditions are often caused by a weakened immune response in the stocks. These health conditions are often intensified by stress, cramped room conditions and other disadvantageous circumstances characterising the modern agricultural production. These conditions and circumstances manifest themselves in many different diseases, which cause problems both from an animal ethical and from a purely financial point of view. The diseases include inter alia digital dermatitis; mastitis; skin, coat and hoof diseases and a weakened milk yield in cattle; diarrhoea and loss of appetite as well as behavioural problems in sows and piglets; coccidiose in poultry and a reduced resistance towards bacterial and viral infections, such as coli and salmonella in all groups of animals.

Furthermore, it is common knowledge and commonly used to include selected vitamins in the vitamin and feed mixtures for animals so as to prevent or cure specific conditions caused by malflourishing animals. Thus, the international Patent Application WO 99/48384 describes a non-human foodstuff comprising biotin and other B vitamins for improving the skin and coat condition of a non-human animal. Vitamin compositions comprising inter alia biotin and vitamin C and E are known from the international Patent Application WO 01/97818; FR Patent Application 2773484 discloses compositions with a therapeutical effect, said compositions inter alia comprising vitamin C and E, organic selenium and trace minerals. The international Patent Application WO 00/25599 describes a vitamin premix for animals which inter alia comprises vitamin A, C and E together with biotin; and EP patent 0 443 743 deals with a vitamin C preparation which also comprises vitamin E, and which can be used for both poultry and stocks. Moreover, the international Patent Application WO 01/17364 deals in particular with vitamin combinations addressing specific health indications in animals, such as flatulence and gastrointestinal diseases; and finally GB patent 595829 is of interest, said patent also dealing with vitamin compositions (vitamin A, C and D and various B vitamins) for animals.

However, a common feature of all these publications is that the use of vitamins, minerals and the like substances as prophylactic or curing solutions of the problems applying to malflourishing animals does not ensure the effect which the individual substances should be able to provide in theory. Therefore, a demand still applies for a vitamin-containing system which can prevent and/or cure the diseases of the animals by being administered in very defined quantities to the animals requiring a treatment. These diseases inflict heavy financial losses on the farmers.

Such a vitamin-containing system is provided by the present invention.

To be more precise, the invention relates to a vitamin-containing system for stabilising the external and the internal immune response of animals, said system including two parts or "kits" to be used successively or separately. The system according to the invention is characterised in that:

| | |
|---|---|
| kit 1 includes | 0.5 to 30% of biotin, |
| | 0.01 to 5% of allicin and |
| | 0 to 10% of crushed radishes (raphanus savitus) mixed in a carrier including oat as stabilizer, and |
| kit 2 includes | 5 to 30% of vitamin E, |
| | 0.1 to 15% of vitamin C, |
| | 0.1 to 1% of selenium, |
| | 0.1 to 10% of allicin and |
| | 0 to 10% of crushed radishes (raphanus savitus) also mixed in a carrier. |

Each kit is mixed in a carrier which suitably includes ground oat, barley and bran. Demineralized water can also be used as carrier.

The two essential ingredients of the above kits are biotin and allicin, which are both commonly known substances. Biotin (hexahydro-2-oxo- 1H-thieno[3,4-d]imidazole-4-pentanoic acid) can also be referred to as vitamin H and is a growth factor existing in small quantities in all live cells. The compound plays a significant part in several native carboxylation reactions, and generally it is bound to proteins or polypeptides. Biotin serves as a cofactor for enzymes critical to the metabolism. A biotin deficiency can result in a deteriorated synthesis and metabolism of long-chained fatty acids which are important for the cutaneous integrity. Biotin reacts with the proteinic substance avidin in raw albumen and turns inactive, and it turned out that animals fed with foodstuff including a high content of albumen develop characteristic skin injuries and disclose a reduced growth. Therefore, a biotin supplement is often used for curing skin and coat diseases in animals. A review of the production and use of biotin can for instance be found in A.F. Wagner & K. Volkers: *Vitamins and Coenzymes* (Wiley, New York, 1964), page 138-159; and in D.B. McCormick, Nutr. Rev. 33 97-102 (1975).

Allicin is the S-allylester ofthio-2-propene-1-sulfinic acid and is a biologically active ingredient of freshly crushed garlic. The substance is an antioxidant and is a native result of the enzyme allicinase acting on alliin, said reaction taking place when the tissue of the garlic bulb is disrupted. Allicin turned out to enhance the immune response and to reduce the curing period of animals by way of an accurate application rate. The isolation, structure and antibacterial activity of the substance has been described in C.J. Cavallito & J.H. Bailey, J. Am. Chem. Soc. 66. 1950-1952 (1944); the synthesis of allicin has been described in US patent 2,508,745. Pharmacological effects of allicin has been described in P.R. Mayeux et al., Agents Actions 25 182 (1988).

It turned out that the good results with respect to prevention and curing by means of the vitamin-containing system according to the invention are conditioned by said vitamin-containing system being administered to the animals according to an accurately determined application rate. However, the application rate is the same whether or not one kit 1 is used alone, kit 2 is used alone or a combination of kit 1 and kit 2 is used and irrespective of the nature of the condition that is to be prevented or cured. The following application rate is always used:

| | |
|---|---|
| The first 7 days | Double dosage compared to the prescribed standard dosage of the ingredient in question. |
| The following 14 days | Ordinary dosage, i.e. a dosage corresponding to the ordinarily prescribed dosage of the ingredient in question |

which results in a total treatment period of 21 days. Subsequently, no further vitamin is to be administered. No other vitamins, minerals or the like substances must be administered at any time during the treatment because these substances highly deteriorate the effect aimed at, and at worst such substances may have the effect that the immune response of the animals is even worse than prior to the treatment.

The treatment using the vitamin-containing system according to the invention starts from a practical, predominantly visual evaluation of deficiency symptoms relating to vitamins in the animals, said evaluation being combined with a diagnosis made by a veterinary with respect to diseases or a disproportion in the stock in question. Then the system to be used in the treatment is chosen in response to the health state evaluated, the diagnosis made and the estimated degree of problems applying. The following guidelines are used for the choice of system:

| | |
|---|---|
| External immune response (hoof, heel, skin, coat etc.) | kit 1 |
| Internal immune response (parasitic and bacterial attacks etc.) | kit 2 |

In many cases the two kits are used separately, but when a health state is revealed which involves both the external and the internal immune response, then kit 1 is initially used followed by an administration of kit 2 or vice versa.

Generally speaking, kit 2 is used in connection with indications of internal diseases, stress and parasitic attacks, in connection with diarrhoea as well as in connection with calving, farrowing and parturitions. Sometimes, it is possible to use substances for reducing the occurrence of micro-organisms in the cowsheds in case an evaluation of the risk of infection and the hygiene dictates such procedures.

It is also possible to involve homoeopathic principles in the treatment.

The vitamin-containing systems according to the invention are advantageously administered in connection with the feeding of the animals. It is in particular possible to use the modern foodstuff mixer vehicles where the vitamin-containing system is mixed directly into the foodstuff and homogeneously distributed therein. The system is not added until maximum 5 minutes, preferably 3 to 4 minutes remain of the total mixing period.

The invention is illustrated in greater detail by means of the following Examples:

### Example 1

A livestock has been treated for digital dermatitis, which is a problematic and cost-intensive disease in cows and heifers. The milk production drops immediately when the cows walk with difficulty due to digital dermatitis, and if the cows are not treated immediately many of them will die and consequently lose their value. Previously, attempts were made at treating the disease by way of hoof trimming or by a medical treatment by means of Hoof® gel, antibiotics or blue vitriol, but without a permanent result.

The animals were treated with kit 1 including biotin for 3 weeks.During the first week, each cow was administered 30 g of biotin, which corresponds to twice the recommended dosage. During the following two weeks, 15 g of biotin was administered to each cow. The administration was made in the foodstuff. The treatment was followed by a treatment with kit 2.

Eight months after completion of the treatment with the system according to the invention, no new cases of digital dermatitis were diagnosed in the livestock.

### Example 2

Milch cows (15 livestocks) have been treated for mastitis and coli infections by initially being administered kit 2 followed by an administration of kit 1. This treatment turned out to be efficient in connection with calvings as well.

One of these livestocks revealed at the beginning 45 cases of mastitis and coli infections (cell count 540,000). After 1 month, the livestock was stable, and after 5 months the quality of life was significantly improved (cell count 155,000). After 12 months, the number of mastitis cases had dropped to 9. During the same period, the number of reproductive disorders dropped from 31 to 2.

The milk production per cow rose on average by 1200 1 in 3 to 4 months.

A treatment by means of kit 2 alone has been carried out on fatteners (10 herds of pigs) in order to combat coli and salmonella infections and coccidiosis. The same treatment has been successfully carried out on two herds of sows in order to combat coli and salmonella infections, black tetter and coccidiosis; and on four herds of piglets in order to enhance the appetite and combat diarrhoea and so as to affect the behaviour of said piglets.

Finally, 15 livestocks of calves (from new-born to 3 months old calves) have been treated in order to combat corona and rotaviral infections, reduce the death-rate and in order to establish a balance of life. Here kit 2 was used as well.

## Claims

1. Vitamin-containing system for stabilising the external and the internal immune response of animals, said system consisting of two parts or "kits" to be used successively or separately, **characterised in that**:
| | |
|---|---|
| kit 1 includes | 0.5 to 30% of biotin, |
| | 0.01 to 5% of allicin and |
| | 0 to 10% of crushed radishes (raphanus savitus) mixed in a carrier including oat as stabilizer, and |
| kit 2 includes | 5 to 30% of vitamin E, |
| | 0.1 to 15% of vitamin C, |
| | 0.1 to 1% of selenium, |
| | 0.1 to 10% of allicin and |
| | 0 to 10% of crushed radishes (raphanus savitus) also mixed in a carrier. |

2. Vitamin-containing system as claimed in claim 1, **characterised in that** the carrier includes ground oat, barley and bran..

3. Vitamin-containing system as claimed in claim 1, **characterised in that** the carrier includes demineralized water.

4. Vitamin-containing system as claimed in any of the claims 1 to 3, **characterised in that** it is administered by using the following dosage:
| | |
|---|---|
| The first 7 days | Double dosage compared to the prescribed standard dosage of the ingredient in question. |
| | |
| The following 14 days | Ordinary dosage, i.e. a dosage corresponding to the ordinarily prescribed dosage of the ingredient in question |
which results in a total treatment period of 21 days.

5. Vitamin-containing system as claimed in any of the claims 1 to 3, **characterised in that** the following guidelines are used with respect to the choice of system:
| | |
|---|---|
| External immune response (hoof, heel, skin, coat etc.) | kit 1 |
| Internal immune response (parasitic and bacterial attacks etc.) | kit 2 |
whereby the two kits can be used successively.
